# FASCICULE DE BREVET EUROPEEN

(11) **EP 0 802 257 B1**
(45) Date de publication et mention de la délivrance du brevet: **21.08.2002**
(21) Numéro de dépôt: 96201064.1
(22) Date de dépôt: 19.04.1996
(51) Int. Cl.: C12N 5/00, C12N 5/10, C12Q 1/00

(54) **Lignée immortalisée de cellules épithéliales du colon humain**
Menschliche immortalisierte Colon-Epithelialzellen
Human colon epithelial immortalized cell lines

(43) Date de publication de la demande: 22.10.1997
(73) Titulaire: SOCIETE DES PRODUITS NESTLE S.A., 1800 Vevey (CH)
(72) Inventeur: Blum, Stéphanie, 1000 Lausanne (CH); Pfeifer, Andrea, 1806 Saint Légier (CH); Troumvoukis, Yvonne, 1074 Mollie-Margot (CH)
(74) Mandataire: Becker Kurig Straus

(56) Documents cités:
- WO-A-89/05862
- DE-A- 3 711 699
- BRITISH JOURNAL OF CANCER, vol. 57, 1988, pages 287-289, XP000604782 BERRY R.D. ET AL: "In vitro culture of human foetal colonic epithelial cells and their transformation with origin minus SV40 DNA"
- GASTROENTEROLOGY, vol. 108, no. 4, 1995, page A321 XP002014543 MOYER M.P. ET AL: "CL- transport characteristics of an immortalised human epithelial cell line (N3) derived from the normal traverse colon"
- IN VITRO CELLULAR & DEVELOPMENTAL BIOLOGY, vol. 23, no. 2, 1987, pages 141-146, XP000604750 MARY PAT MOYER ET AL: "Phenotypic changes and gene expression in human colon mucosal epithelial cells upon transfection of a SV40 DNA-GTP recombinant"
- SCIENCE, vol. 224, 1984, pages 1445-1447, XP000604741 MARY PAT MOYER ET AL: "Human colon cells: culture and in vitro transformation"
- PROCEEDINGS OF THE NATIONAL ACADEMY OF SCIENCES OF USA, vol. 78, no. 9, 1981, WASHINGTON US, pages 5588-5592, XP002014544 BETTGER W.J. ET AL: "Rapid clonal growth and serial passage of human diploid fibroblasts in lipid-enriched synthetic medium supplemented with epidermal growth factor, insulin and dexamethasone"
- R. IAN FRESHNEY: "CULTURE OF ANIMAL CELLS, A MANUAL OF BASIC TECHNIQUE" 1987 , ALAN R. LISS, INC. , NEW YORK XP002014546 * page 74 - page 84 *
- FASEB JOURNAL FOR EXPERIMENTAL BIOLOGY, vol. 6, 1992, BETHESDA, MD US, pages 2726-2734, XP002014545 A. BATEN ET AL: "Long-term culture of normal human colonic epithelial cells in vitro"
- "CATALOGE OF CELL LINES AND HYBRIDOMAS" 1988 , AMERICAN TYPE CULTURE COLLECTION , ROCKVILLE, MARYLAND XP002014547 * page 146 * * page 174 * * page 344 *
- THE AMERICAN JOURNAL OF SURGERY, vol. 169, 1995, pages 190-196, XP000604655 STAUFFER J.S. ET AL: "Development and characterization of normal colonic epithelial cell lines derived from normal mucose"
- INTERNATIONAL ARCHIVES OF ALLERGY AND IMMUNOLOGY, vol. 107, 1995, pages 396-397, XP000604653 SANDERSON I.R. ET AL: "Establishment of a human fetal small intestinal epithelial cell line"
- JOURNAL OF THE NATIONAL CANCER INSTITUTE, vol. 58, no. 2, 1977, pages 209-214, XP000604656 FOGH J. ET AL: "Absence of HeLa cell contamination in 169 cell lines derived from human tumors"

## Description

La présente invention a pour objet une nouvelle lignée immortalisée de cellules épithéliales du colon humain, une méthode pour obtenir cette lignée, ainsi que toute utilisation de cette lignée, notamment dans des procédés d'identification d'agents mutagéniques, toxiques ou bénéfiques pour le métabolisme des cellules du tractus intestinal.

### Etat de la technique

Depuis de nombreuses années, des efforts sont entrepris pour développer des lignées cellulaires humaines adaptées à l'étude des maladies humaines, comme les infections, les inflammations ou les cancers, par exemple. Parmi les cellules impliquées souvent dans l'apparition de maladies, on peut compter les cellules épithéliales, en particulier les cellules épithéliales du tractus intestinal qui sont sensibles à l'environnement du corps humain, et notamment aux conditions de son alimentation.

Les cellules épithéliales se distinguent des autres cellules du corps humain par l'expression de composés ou structures retrouvés principalement dans les cellules épithéliales, comme par exemple des cytokeratines (Moll et al., Cell, 31, 11-24, 1982), des connections entre les cellules (Gumbiner et al., Cell, 69, 385-387, 1992), la phosphatase alcaline spécifique de l'intestin (Dudeja et al., Gastroenterology, 105, 357-366, 1993), des cytochromes P450 (Mercurio et al., Biochem. Biophy. Research Communications, 210, n°2, 350-355, 1995; McKinnon et al., Gut, 36, 259-267, 1995), des enzymes impliquées dans l'oxydation cellulaire (Cu/Zn-superoxide dismutase, une glutathion péroxidase et catalase; Albers et al., Toxicology and Applied Pharmacology, 109, 507-513, 1991) et/ou dans la détoxification d'électrophiles (glutathion-S-transférase et quinone réductase; Chenevix- Trench et al., Carcinogenesis, 16, n°7, 1655-1657, 1995), et la vimentine (Richard et al., Arch. Dematol. Res., 282, 512-515, 1990),

En outre, les cellules épithéliales du tractus intestinal humain sont capables d'adhérer *in-vitro* à des bactéries lactiques (Bernet et al., Gut, 35, 483-489, 1994; US 5.032.399)

Enfin, les cellules épithéliales du colon humain se distinguent également des autres cellules épithéliales humaines par l'expression de composés ou structures retrouvés principalement dans les cellules épithéliales du colon humain, comme par exemple des villosités de surface (Friedrich et al., Bioassays, 12, n°9, 403-408, 1990), la sucrose isomaltase (Gibson et al., Gut, 35, 791- 797, 1994; Paul et al., Am. Physiol. Soc., C266-C278, 1993), certains antigènes du complexes majeurs d'histocompatibilités de classe II (Mayer et al., Gastroenterology, 100, 3-12, 1991), et la dipeptidylpeptidase IV (DPPIV; Hauri et al., J. Cell. Biol., 101, 836-851, 1985).

Moyer et al. (Gastroenterology, 108, A321, 1995) et Stauffer et al. (Gastroentrology, 106, A273, 1994) décrivent l'isolement et la caractérisation de cellules épithéliales non-transformées du colon, et en particulier la lignée NCM460(N3) qui exprime certains marqueurs épithéliaux.

La préparation d'une lignée de cellules épithéliales du colon humain peut être réalisée par sélection de cellules humaines cancéreuses. Stauffer et al. décrivent ainsi la sélection de deux lignées NCM356 et NCM425 qui comprennent la mutation p53 et qui expriment notamment l'antigène tumoral CEA (The American journal of surgery, 169, 190-196, 1995). Ces cellules sont a un stade de transformation cancéreuse préliminaire, et présentent un intérêt pour l'étude du développement de la transformation cancéreuse des cellules épithéliales.

On connait aussi d'autres lignées de cellules épithéliales du colon humain isolées à partir d'adénome du colon humain, comme par exemple les lignées CaCO-2 (ATCC HTB37; Fogh et al., J. Natl. Cancer Inst., 58, 209-214, 1977) et HT29 (ATCC HTB38; Fogh et al., Human tumor Cells In Vitro, 145-159, 1975)

Il est aussi possible d'immortaliser des lignées cellulaires normales, c'est à dire les rendre capables de se multiplier indéfiniment. En effet, les cellules normales ne survivent pas à plus de quelques dizaines de passages. Pour cela, on recours généralement à des techniques de transfection des cellules, à l'aide de vecteurs spécialement adaptés, comme le vecteur SV40 comprenant une séquence de l'antigène large T (R.D. Berry et al., Br. J. Cancer, 57, 287-289, 1988), ou encore un vecteur comprenant des séquences d'ADN du papillomavirus humain (US5376542).

Sanderson et al. ont ainsi immortalisé des cellules normales de l'intestin grêle foetal (Int. Arch. Allergy Immunol., 107, 396-397, 1995). Cependant, ces cellules restent encore physiologiquement éloignées des cellules normales de l'adulte, du fait de l'absence ou de la relative faible abondance de marqueurs spécifiques des cellules normales, comme les connections entre cellules épithéliales, par exemple.

A ce jour, il n'a pas été rapporté une immortalisation de cellules épithéliales normales du colon humain adulte. Même si les techniques d'immortalisation sont connues, il est encore bien difficile de trouver les conditions optimales pour immortaliser une cellule humaine de façon a ce qu'elle conserve ses caractéristiques originelles, et cela sans présenter de signes d'une transformation cancéreuse.

Une des conditions d'immortalisation de cellules humaines est en particulier le milieu de culture. Les milieux de culture décrits dans la littérature sont chacuns spécifiques d'un type cellulaire et ne peuvent pas aisément être appliqués à d'autres types cellulaires. A titre d'exemple, on peut citer les milieux sans sérum décrits par Gibson et al. (Gut, 35, 791-797, 1991), Pfeifer et al. (Proc. Natl. Acad. Sci. USA, 90, 5123-5127, 1993) et Kulkani et al. (Carcinogenesis, 16 n°10, 2515-2521, 1995). WO 97/18296, qu'est un document selon l'article 54 (3) CBE, décrit des méthodes d' obtenir une culture primaire de cellules épithéliales humaines en utilisant un système de culture de tissu de co-culture des fibroblastes. Les procédés peuvent être utilisés pour obtenir des cultures de cellules épithéliales normales, précancéreuses et malignes. Des procédés permettant de dériver des souches de cellules épithéliales substantiellement pures d' une culture primaire et pour immortaliser des souches cellulaires pour obtenir des lignes cellulaires..

Le but de l'invention vise à fournir de nouvelles lignées de cellules épithéliales du colon humain qui se rapprochent génétiquement et physiologiquement des cellules épithéliales normales du colon humain, au point qu'il est difficile de les différencier. En particulier, ces lignées n'expriment pas de marqueurs tumouraux. Ces lignées expriment en outre de nombreux marqueurs spécifiques retrouvés sur des cellules épithéliales normales du colon humain.

### Résumé de l'invention

A cet effet, l'invention concerne toute lignée immortalisée de cellules épithéliales du colon humain, immortalisée à l'aide d'un virus recombinant, la dite lignée étant capable de se multiplier durant au moins 60 passages successifs, dépourvue de marqueurs tumoraux, exprimant des marqueurs métaboliques spécifiques des cellules épithéliales humaines non-immortalisées et des marqueurs métaboliques de différenciation spécifiques des cellules épithéliales non-immortalisées du colon humain, capable d'adhérer *in-vitro* à la souche de bactérie lactique CNCM I-1225 et capable d'être obtenue selon le procédé d'immortalisation décrit ci-après, sous réserve que les lignées de cellules soient exclues qui sont obtenues par un procédé pour obtenir une lignée immortalisée de cellules épithéliales humaines substantiellement pure, comprenant :
(a) mettre à disposition d'une culture primaire de cellules épithéliales humaines obtenue en utilisant un système de co-culture de fibroblastes, comprenant une première population de cellules comprenant une culture de fibroblastes humains sur un substrat dans un milieu approprié, un support du type d'une membrane, le support étant arrangé sur la culture de fibroblastes et une deuxième, hétérogène population de cellules appliquée sur le support et comprenant des cellules épithéliales humaines,
(b) ensemencer sur une plaque une troisième population de cellules comprenant une culture de fibroblastes humains sur un deuxième substrat dans un milieu approprié,
(c) détacher sélectivement des cellules non-épithéliales du substrat en incubant la co-culture avec un agent détachant approprié pendant une période de temps suffisante pour détacher des cellules non-épithéliales du substrat sans détacher une quantité substantielle de cellules épithéliales,
(d) arranger le support sur la culture des fibroblastes obtenue par l'étape (b), afin qu'une souche de cellules épithéliales substantiellement pure soit maintenue sur le support, et
(e) immortaliser ladite souche de cellules épithéliales substantiellement pure avec au moins un vecteur rétroviral ayant une cassette d'expression comprenant un molécule d'acide nucléique codant pour un polypeptide de papillomavirus choisi dans le groupe formé par E6, E7 et par des combinaisons de ceux-ci, et des séquences de contrôle qui dirigent la transcription du molécule d'acide nucléique, afin que la séquence puisse être transcrite et traduite dans la lignée cellulaire transfectée.

L'invention a également pour objet un milieu de culture sans sérum adapté à la culture et à l'immortalisation de cellules épithéliales du colon humain. Ce milieu comprend les constituants habituellement retrouvés dans les milieux sans sérum utilisés pour cultiver les cellules animales, comme par exemple des sels inorganiques, du glucose, du pyruvate de sodium, des acides aminés, de la BSA, de la phosphoéthanolamine, de l'éthanolamine, des vitamines, et des hormones. Ce milieu comprend en outre une combinaison nouvelle de certains de ses constituants, à savoir des traces d'oligoéléments, des vitamines consistant en la vitamine C et l'acide rétinoique, et des hormones consistant en la triiodothyrodine, la déxamethasone, l'hydrocortisone, l'extrait de la glande pituaire bovine, l'insuline, l'EGF et la transférrine.

Un autre aspect de l'invention concerne un nouveau procédé d'immortalisation de cellules épithéliales du colon humain, dans lequel on prépare une culture de cellules épithéliales primaires issues du colon humain, on infecte la culture avec un virus recombinant, et on cultive les cellules immortalisées dans le milieu de culture sans sérum décrit précédemment.

Un autre aspect de l'invention concerne un procédé pour identifier l'effet mutagène, toxique ou bénéfique d'un agent sur le métabolisme des cellules du tractus intestinal, dans lequel, *in-vitro*, (1) on fait réagir, on cultive ou on met en contact avec une culture comprenant une lignée cellulaire décrite précédemment, un agent soupçonné d'être un agent mutagène, toxique ou bénéfique pour le métabolisme des cellules du tractus intestinal, et (2) on détermine ou on mesure les effets dudit agent sur ladite lignée cellulaire.

L'invention concerne aussi un kit de diagnostique comprenant les cellules épithéliales immortalisées du colon humain décrite précédemment, le milieu de culture décrit précédemment, et des réactifs pour déterminer une réponse métabolique desdites cellules à des agents mutagéniques, toxiques ou bénéfiques pour le métabolisme des cellules du tractus intestinal.

Enfin, l'invention a aussi pour objet toutes utilisations des lignées cellulaires décrites précédemment, dans des procédes *in-vitro* d'identification d'agents mutagéniques, toxiques ou bénéfiques pour le métabolisme des cellules du tractus intestinal, ainsi que toutes utilisations desdites lignées pour la préparation d'un agent pharmaceutique actif.

### Description des figures

Figure 1: vecteur pLXSHD+SV40+ utilisé pour immortaliser les cellules épithéliales du colon humain.

Figure 2: expression de l'activité de la phosphatase alcaline par différentes lignées cellulaires issues de cellules épithéliales du colon humain.

Figure 3: pourcentage de cellules épithéliales immortalisées selon l'invention qui expriment des antigènes du CMH (HLA) de classe II en présence d'interféron gamma, en fonction du temps.

### Description détaillée de l'invention

Dans le cadre de la présente invention, les expressions "cellules normales", "cellules primaires" ou "cellules non-immortalisées" désignent des cellules épithéliales du colon humain que l'on peut prélever sur le colon d'un adulte sain ne présentant pas de déficiences physiologiques ou génétiques handicapantes, et que l'on peut cultiver pendant un temps limité sans qu'elles perdent leurs caractéristiques de différenciation originelle.

Par contre, l'expression "cellules immortalisées" désigne des cellules qui ont subi une manipulation génétique, par l'intermédiaire d'une construction d'ADN, qui les rend capables de se multiplier indéfiniment, c'est à dire à plus de 60 passages, Les cellules cancéreuses sélectionnées par Stauffer et al., ou les lignées CaCO-2 et HT29 ne sont ainsi pas considérées comme des cellules ayant été immortalisées selon la présente invention.

De même, le mot "passage" désigne le processus consistant à prendre un aliquôt d'une culture confluente d'une lignée cellulaire, à ensemmencer un milieu frais, et à cultiver la lignée jusqu'à confluence ou saturation. Les lignées cellulaires sont ainsi cultivées traditionnellement par passages successifs dans des milieux frais. Il faut noter que les lignées cellulaires peuvent perdre leurs caractéristiques de différentiation originelle après plusieurs passages successifs. Il est donc extrêmement avantageux de pouvoir disposer d'une lignée dont ses caractéristiques sont conservées, même après de nombreux passages, de préférence au moins 60 passages.

Enfin, l'expression "caractéristiques de différentiation originelle" désigne à la fois les marqueurs retrouvés spécifiquement sur les cellules épithéliales humaines et les marqueurs de différentiation retrouvés spécifiquement sur les cellules épithéliales de colon humain.

Les lignées immortalisées de cellules épithéliales du colon humain selon l'invention n'expriment pas de marqueurs tumoraux, c'est à dire ne présentent pas de gènes cancérigènes ou n'expriment pas d'ARN messagers (ARNm), protéines, et/ou structure cellulaires différentiées caractéristiques de la transformation des cellules en cellules tumorales. On peut détecter la présence de ces marqueurs par hybridation ou PCR de leur ADN avec une sonde spécifique, par des anticorps, et/ou par microscopie électronique, par exemple. La présence d'un marqueur dans une cellule ne signifie pas que ladite cellule est susceptible de conférer un cancer après quelques mois à une souris sans défense immunitaire, mais traduit plutôt un état transformé cancereux des cellules par rapport aux cellules originelles dont elles sont issues.

De préférence, la lignée selon la présente invention ne présente pas la mutation p53 (Lavigeur et al., Mol. Cell. Biol., 9, 3982-3991, 1989; Donehower et al., Nature, 356, 215-221, 1992), l'antigène du carcinome embryonnaire (CEA; Stauffer et al.), et/ou le gène APC (adenomatous polyposis coli; Hargest et al., Gut, 37, 826-829, 1995).

Les lignées cellulaires épithéliales selon l'invention expriment par contre des marqueurs métaboliques spécifiques des cellules épithéliales humaines normales. Ces marqueurs peuvent être un ARN messager (ARNm), une protéine, et/ou une structure cellulaire différentiée, que l'on retrouve généralement uniquement dans la majorité des cellules épithéliales humaines, pouvant provenir de la peau, de l'oeil, du tractus intestinal, ou du foie, par exemple. Les cellules épithéliales humaines selon l'invention peuvent donc exprimer au moins deux marqueurs retrouvés dans différents types de cellules épithéliales. De préférence, les cellules selon l'invention expriment lesdits marqueurs choisis dans le groupe formé par au moins des cytokeratines, des connections entre les cellules (appellées encore en anglais "tight junctions"), la phosphatase alcaline, des cytochromes P450, la glutathion-S-transférase (GST), la quinone réductase (QR), la Cu/Zn-superoxide dismutase (SOD), la glutathion péroxidase (GP) et la catalase (CA).

Les lignées selon l'invention peuvent donc exprimer des enzymes impliquées dans l'oxydation cellulaire (SOD, GP et CA) et/ou la détoxification d'électrophiles (GST et QR). Ces lignées sont donc particulièrement adaptées à l'étude des phénomènes d'inflammations ou irritations des muqueuses du colon humain.

Les lignées cellulaires selon l'invention expriment aussi des marqueurs métaboliques de différenciation qui sont spécifiques des cellules épithéliales non-immortalisées du colon humain. Ces marqueurs peuvent être un ARNm, une protéine, ou une structure cellulaire différentiée que l'on retrouve uniquement dans les cellules épithéliales du colon. De préférence, les lignées selon l'invention, expriment comme marqueur métabolique de différenciation au moins deux marqueurs choisis dans le groupe formé par des villosités de surface, la sucrose isomaltase, les complexes majeurs d'histocompatibilités de classe II, et la dipeptidylpeptidase IV.

De même, les lignée selon l'invention expriment de préférence les antigènes HLA-DR et HLA-DP des complexes majeurs d'histocompatibilités de classe II (CMH II), et n'expriment pas l'antigène HLA-DQ, en présence d'interféron gamma humain.

Les lignées selon l'invention doivent également être capables d'adhérer *in-vitro* la souche de bactérie lactique CNCM-1225. Pour cela, il suffit d'étaler une culture de bactérie lactique sur une culture confluente d'une lignée selon l'invention, de laver la culture confluente, puis de mesurer le nombre de bactéries adhérantes aux villosités desdites lignées. De préférence, une lignée selon l'invention est capable d'adhérer *in-vitro* la souche de bactérie lactique CNCM-1225 à raison d'au moins 80 bactéries pour 100 cellules du colon selon l'invention, notamment 80-200.

L'invention concerne aussi en particulier une lignée immortalisée selon l'invention qui a été déposée auprès de la Deutsche Sammlung Von Mikroorganismen und Zellkulturen, Mascheroder Weg 1b, D-38124 Braunschweig, Allemagne, le 16 avril 1996, où elle a reçue le numéro de dépôt DSM ACC2258.

Les lignées épithéliales du colon humain selon l'invention conservent avantageusement leurs caractéristiques de différentiation originelle même après de nombreux passages, notamment au moins 40-80 passages. On peut toutefois noter après le 40ième passage, voire même avant, un changement du caryotype des lignées suite à la perte de fragments de chromosomes. Néanmoins, ces lignées conservent généralement quant même un exemplaire intacte de chaque paire de chromosomes, ce qui leur permet de conserver également l'expression de leurs caractéristiques de différentiations originelles.

Un autre aspect de la présente invention concerne un milieu de culture sans sérum adaptée à la culture et à l'immortalisation de cellules épithéliales du colon humain selon l'invention. Ce milieu de culture est particulièrement adapté pour maintenir indéfiniment, lors des passages successifs, les caractéristiques de différentiation originelle des lignées selon l'invention. Si d'autres milieux de cultures sont utilisées, les lignées selon l'invention pourraient ainsi perdre leurs caractéristiques de différentiation originelle à la suite de quelques passages successifs, par exemple 5-10 passages. Ce milieu est également indispensable pour immortaliser avec succès des lignées selon l'invention. La supression d'un de ses constituants caractéristiques aboutit ainsi généralement à l'échec de l'immortalisation de cellules épithéliales du colon selon l'invention.

Ce milieu peut comprendre tous les constituants habituellements retrouvés dans les milieux de culture de cellules animales sans sérum, à savoir des sels inorganiques, du glucose, un tampon (HEPES, par exemple: Biofluids), du pyruvate de sodium, des acides aminés, de la BSA, de la phosphoéthanolamine, de l'éthanolamine, des vitamines, et des hormones, par exemple. Les milieux de cultures commerciaux, comme le milieu DMEM (Biofluids Inc, USA), peuvent ainsi servir de base pour la préparation de ce milieu.

L'originalité du milieu selon l'invention réside dans une combinaison nouvelle de certains de ses constituants, tandis que les autres composés habituellement nécéssaires peuvent varier dans les limites connues de l'homme du métier. Ces constituants indispensables sont des traces d'oligoéléments, des vitamines consistant en la vitamine C et l'acide rétinoique, et des hormones consistant en la triiodothyrodine, la déxamethasone, l'hydrocortisone, l'extrait de la glande pituaire bovine, l'insuline, l'EGF et la transférrine.

Ce milieu de culture peut ainsi comprendre 1-100 nM d'oligoéléments, 10-1000 nM d'acide rétinoique, 10-1000 nM de triiodothyrodine (T3), 0,1-50 nM de déxamethasone, 1-100 nM d'hydrocortisone, 1-100 µg/mlde vitamine C, 1-100 µg/ml d'extrait de la glande pituaire bovine, 0,1-50 µg/ml d'insuline, 0,1-50 ng/ml d'EGF et 0,1-100 µg/ml de transférrine.

Parmi les oligoéléments, on peut choisir les composés choisis dans le groupe formé par le selenium, le manganèse, le silicate, le molybdène, le vanadium, le nickel, l'étain, et leurs sels, par exemple.

Parmi les sels inorganiques habituellement inclus dans le milieu, on peut choisir des sels dans le groupe formé par le chlorate de sodium, le chlorate de potassium, le sulfate de potassium, le chlorate de calcium, le phosphate de sodium di-basique, le bicarbonate de sodium, le nitrite ferrique, le métévanadate d'ammonium, le molybdate d'ammonium, le sulfate cuprique, le chlorate de magnésium, le chlorate de manganèse, le sulfate de nickel, l'acétate de sodium, le pyruvate de sodium, le sélénite de sodium, le silicate de sodium, le chlorate d'étain, et le sulfate de zinc, par exemple.

En particulier, on peut prendre garde à ce que le milieu contienne moins de 80 µM de calcium sous forme de sels inorganiques, de façon à inhiber le développement de fibroblastes contaminants.

De même, parmi les autres vitamines habituellement incluses dans le milieu, on peut choisir des vitamines choisies dans le groupe formé par le D-pantothénate de calcium, le chlorate de choline, l'acide folique, l'inositol, la nicotinamide, la pyridoxine, la riboflavine, la thiamine, la biotine et le cyanocobalamine, par exemple.

Enfin, un milieu de culture permettant d'obtenir avec succès des lignées selon l'invention présente de préférence la composition B50 définie au tableau 1 ci-après.

Un autre aspect de l'invention concerne un procédé d'immortalisation de cellules épithéliales du colon humain, dans lequel on prépare une culture de cellules épithéliales primaires issues du colon humain, on infecte la culture avec un virus recombinant, on cultive les cellules immortalisées dans le milieu de culture sans sérum décrit précédemment.

Pour cela, on met en oeuvre de préférence les étapes suivantes:
(1) on obtient un échantillon de tissus épithéliaux d'un colon humain;
(2) on prépare cette échantillon en vue de sa culture *in-vitro*;
(3) on ensemmence les cellules épithéliales dans un milieu de culture sans sérum et sur des plaques de culture comprenant un revêtement qui facilite l'attachement des cellules et leur croissance;
(4) on change le milieu autant de fois que nécéssaire pour optimiser la croissance confluente;
(5) on infecte les cellules avec un virus recombinant;
(6) et on cultive les cellules immortalisées dans le milieu de culture sans sérum décrit précédemment.

Plus en détail, l'étape 1) concerne l'obtention d'échantillons de cellules du colon d'individus normaux lors d'actes de chirurgie du tractus intestinal. A l'étape 2), on peut laver l'échantillon dans le milieu de culture, le couper en morceaux, séparer la partie épithéliales des autres tissus par des moyens physiques et/ou chimiques. Par example, on peut placer les morceaux de tissus dans une solution comprenant environ 0,5% de trypsine et 0,1% d'EDTA pendant un temps suffisant pour arriver à séparer les cellules, puis ajouter un inhibiteur de la trypsine pendant quelques minutes, et enfin ajouter du milieu de culture, de préférence celui décrit précédemment.

A l'étape 3), on peut ensemmencer les cellules épithéliales sur des plaques dans un milieu de culture sans sérum, de préférence celui décrit précédemment. Les plaques de culture présentent de préférence un revêtement constitué d'une solution de fibronectine, de BSA et de collagène du type 1 (voir Lechner et al., J. Tissue Cult. Meth., 9, 43-48, 1985).

A l'étape 4), le milieu de culture contenant les cellules épithéliales est changé autant de fois que nécéssaire pour optimiser une croissance confluente. De préférence, le milieu est remplacé tous les deux jours. Après avoir atteind une confluence de l'ordre de 90% de la surface disponible, ce qui intervient généralement 10 à 14 jours après l'ensemmencement, les cellules sont séparées par des traitements dans des solutions de trypsine et d'EDTA.

Les cellules séparées sont transférées à l'étape 5) dans un milieu d'infection, par exemple celui décrit précédemment, sur des plaques de culture ayant de préférence le revêtement décrit ci-dessus. Les cellules sont ensuites infectées d'une manière classique par un virus recombinant. De nombreuses techniques de transfection sont à la disposition de l'homme du métier. A titre d'exemple on peut citer les techniques décrites dans WO96/07750, par Claudia Chen et al. (Mol. and Cell. Biol., 7, 2745-2752, 1987) ou par Wilson et al. (Analytical Biochemistry, 226, 212-220, 1995).

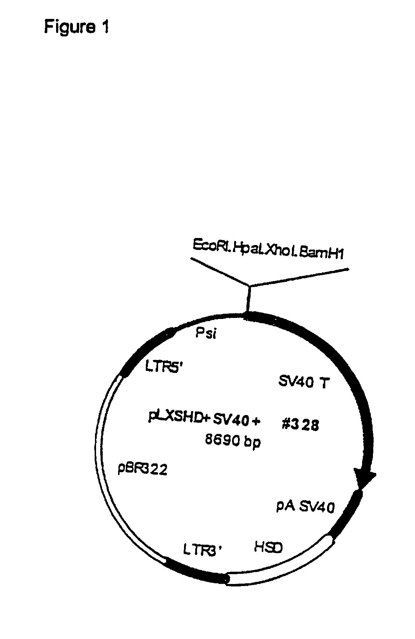

De préférence on utilise un virus recombinant SV40 comprenant l'Antigène T (Ag-T), une origine de réplication fonctionnelle chez l'homme et une partie du rétrovirus MuLV. A titre d'exemple, on peut ainsi utiliser la construction présentée à la figure 1 dont la séquence est disponible dans la banque de donnée GeneBank (n° accession M64753; Stockshlaeder et al., Human Gen. Therapy, 2, 33-39, 1991). D'autres vecteurs appropriés peuvent être aussi facilement construits par l'homme du métier à partir de vecteurs disponibles commercialement comprenant par exemple le gène codant pour Ag-T, un gène de sélection et/ou une origine de réplication humaine. A titre d'exemple, on peut citer les plasmides ATCC37460 et ATCC37640 qui contiennent le gène codant pour Ag-T.

A l'étape 6) on transfert les cellules épithéliales dans un milieu de croissance frais, sur des plaques de culture comprenant de préférence le revêtement décrit ci-dessus.

Connaissant les propriétés nouvelles et originales des lignées de cellules épithéliales selon l'invention, on peut envisager leur application dans des études immunologiques, pharmacologiques et toxicologiques

Les lignées selon l'invention sont ainsi particulièrement adaptées pour cribler des agents mutagéniques, toxiques ou bénéfiques pour le métabolisme des cellules du tractus intestinal, par exemple dans un procédé dans lequel (1) on fait réagir, on cultive ou on met en contact avec une culture comprenant une lignée cellulaire selon l'invention, un agent soupçonné d'être un agent mutagène, toxique ou bénéfique pour le métabolisme des cellules du tractus intestinal, et (2) on détermine ou on mesure les effets dudit agent sur ladite lignée cellulaire.

On peut donc envisager également de préparer un kit de diagnostique comprenant les cellules épithéliales selon l'invention, le milieu de culture selon l'invention, et des réactifs pour déterminer une réponse métabolique desdites cellules à des agents mutagéniques, toxiques ou bénéfiques.

Les lignées selon l'invention sont aussi adaptées à l'expression de protéines recombinantes. Les méthodes de transfection d'ADN étranger sont maintenant bien connues de l'homme du métier (voir par exemple WO 94/05472)

Les lignées selon l'invention ont aussi une utilité potentielle dans la thérapie génetique *ex-vivo*. Ces lignées pourraient constituer en effet un outil adéquat pour développer des cellules recombinantes exprimant des gènes d'intérêt en vue d'une application thérapeutique. Un avantage présenté en plus par les lignée selon l'invention, est qu'elles ne sont pas exposées à un sérum animal, ce qui réduit considérablement les risques de contaminations potentiels par des virus ou autres agents pathogènes.

La présente invention est décrite plus en détail ci-après à l'aide du complément de description qui va suivre, qui se réfère à des exemples de préparations de lignées cellulaires selon l'invention. Ces exemples sont précédés d'une description des milieux de culture utilisées, ainsi que d'un exemple comparatif. Il va de soi, toutefois, que ces exemples sont donnés à titre d'illustration de l'objet de l'invention dont ils ne constituent en aucune manière une limitation. La culture de lignée cellulaire, la préparation de vecteurs SV40, la transfection et l'analyse de l'expressions des marqueurs sont, en l'absence de précisions contraires, effectuées selon les protocoles décrits dans l'ouvrage de Sambrook *et al.* (Molecular Cloning, A Laboratory Manual, Cold Spring Harbor Laboratory Press, U.S.A., 1989). Les pourcentages sont donnés en poids, sauf indication contraire.

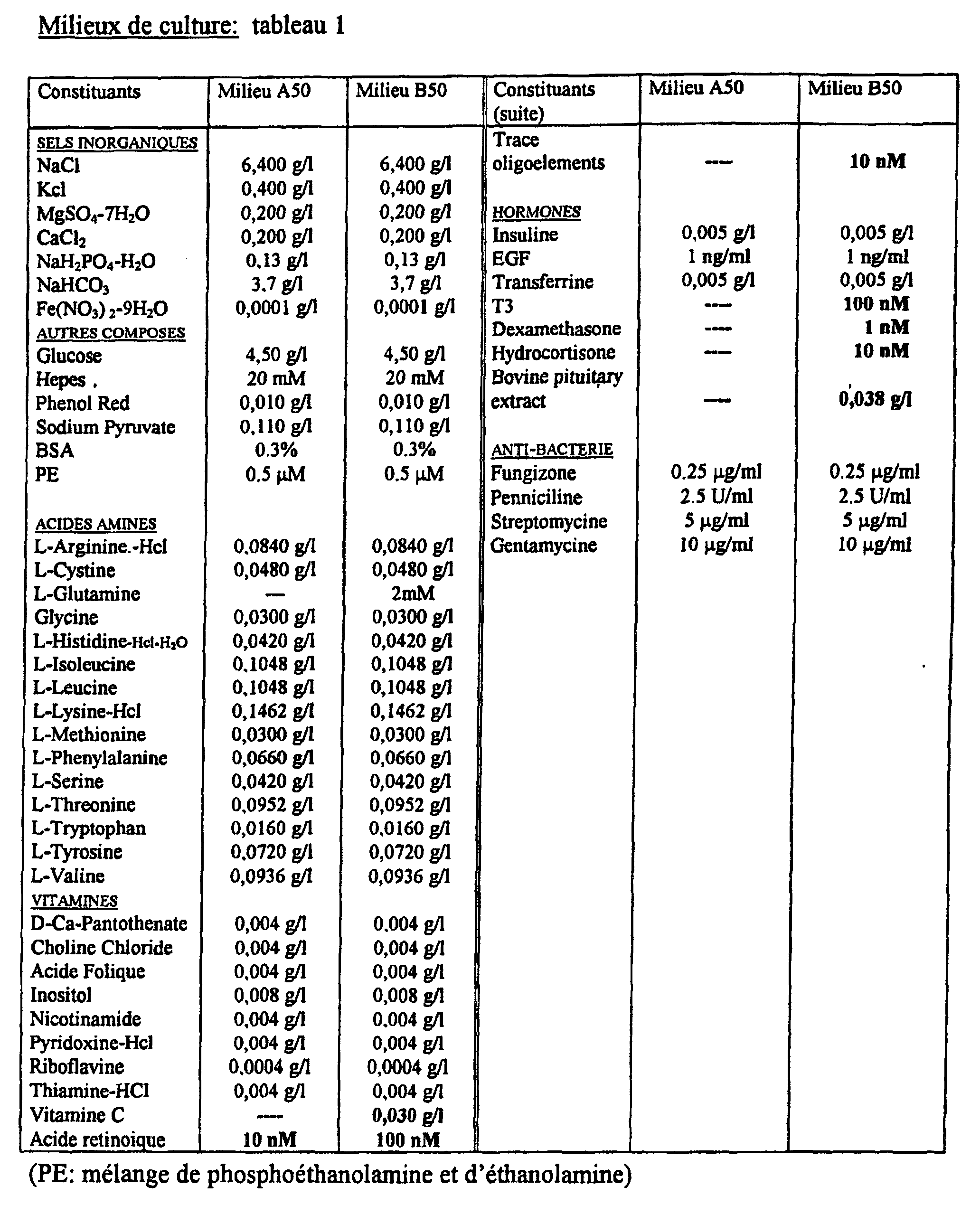

### EXEMPLE COMPARATIF

On utilise le vecteur pLXSHD+SV40+ décrit par Stockshlaeder et al (GeneBank, n° accession M64753; Stockshlaeder et al., Human Gen. Therapy, 2, 33-39), qui est représenté à la figure 1, et qui comprend le gène codant pour l'antigène large T, le gène psi, le marqueur de sélection histidinol dehydrogenase (HSD), et le promoteur LTR.

On prépare les virus SV40 selon une version modifiée du protocole de Lynch et Miller (J. Virol. , 65, 3887-3890, 1991). Pour cela, on cultive les lignées cellulaires ecotropique Psi2 (Mann et al., Cell, 33, 153-159, 1983) et amphotropique PA317 (ATCC CRL9078) dans le milieu DMEM (Dulbecco, USA) comprenant 10% de serum foetal de veau, à 37°C et sous une atmosphère comprenant 5% de CO₂. On transfecte classiquement ces lignées séparément en utilisant 250mM de CaCl2 et 10 µg du vecteur du vecteur pLXSHD+SV40+, on les soumet à un traitement à la trypsine après 48h d'incubation, on les mélange en quantité égale, et on incube le tout à 37°C, sous une atmosphère comprenant 5% de CO₂. Après croissance jusqu'à 70% de confluence, on récolte les virus dans le milieu sans sérum PC-1 (Ventrex, USA). Après filtration (0,45µm, Micropore), on détermine la quantité de virus sur des cellules NIH 3T3 (ATCC CRL 1658).

Des cellules primaires du colon ont été obtenues suite à la biopsie d'une femme de 69 ans due à l'apparition d'un diverticul sigmoidal. On lave l'échantillon dans le milieu de culture DMEM, on le coupe en morceaux, on sépare la partie épithéliales des autres tissus, on ensemmence les cellules épithéliales sur des plaques dans le milieu DMEM. Les plaques de culture ont été préincubées dans une solution de 500ml comprenant 5 mg de fibronectine humaine (Sigma), 5 ml de Vitrogen 100 (Collagen Corp. Palo Alto, CA, USA) 50 ml d'une solution de BSA à 0,1% (Biofuids) et le reste de milieu DMEM. Le milieu de culture contenant les cellules épithéliales est changé autant de fois que nécéssaire pour optimiser une croissance confluente.

Après avoir atteind une confluence de l'ordre de 90% après 2 semaines de culture, les cellules sont séparées classiquement par traitements dans des solutions de trypsine et d'EDTA. Les cellules séparées sont transférées sur des plaques de culture ayant le revêtement décrit ci-dessus, et dans le milieu A50 décrit ci-dessus.

Les cultures sont ensuites infectées en présence de 8µg/ml de polybrene et un haut titre de virus recombinant SV40 (10⁵-10⁷ CFU) comprenant l'Antigène T (Ag-T) préparé comme décrit ci-dessus. Après 2 h d'incubation, on lave les cultures dans du PBS et on ajoute le milieu de croissance frais A50.

Les cellules présentent par la suite malheureusement une croissance tellement lente, qu'il s'avère impossible de sélectionner un clone présentant une croissance comparable à celle de cellules épithéliales du colon humain.

### EXEMPLE 1

On infecte les cellules épithéliales du colon humain comme décrit à l'exemple comparatif ci-dessus, à la différence près que le milieu A50 est remplacé par le milieu B50 décrit au tableau 1 ci-dessus.

Après 2 h d'incubation avec le virus, on lave les cultures dans du PBS, on ajoute le milieu de croissance frais B50, et on cultive les cellules par passages successifs dans du milieu frais B50, en prenant garde à chaque passage de séparer les cellules par un traitement dans une solution comprenant 0,02% de trypsine, 1% de polyvinylpyrolidine et 0,02% d'éthylène glycol dans le tampon HEPES (Biofluids).

Par observation de la taille des colonies cellulaires se développant sur les plaques de culture, on sélectionne celles qui se développent rapidement. On a ainsi pu sélectionner 6 lignées immortalisées de cellules épithéliales du colon humain, dont une a été déposée auprès de la Deutsche Sammlung Von Mikroorganismen und Zellkulturen (DSM), Mascheroder Weg 1b, D-38124 Braunschweig, Allemagne, le 16 avril 1996, où elle a reçue le numéro de dépôt DSM ACC2258.

### 1. Analyse du caryotype de la souche DSM ACC2258.

On prépare 4 caryotypes de la souche DSM ACC2258 prise au 40^{ième} passage (méthode décrite dans le manuel "Human cytogenetics, Edts: Rooney DE, Czepulkowski BH, IRL Press, Oxford, 1986", incorporé par référence). Les résultats montrent que 6 chromosomes différents ont été perdus ou endommagés. La lignée conserve donc intacte dans son génome un chromosome de chaque paire de chromosomes. Le sexe de la lignée est XO/XX. Il est à noter que la perte d'un exemplaire d'un chromosome n'est pas révélateur d'une transformation cancéreuse de ladite lignée.

### 2. Tumorigenicité de la souche DSM ACC2258

On injecte 10⁷ cellules de la lignée DSM ACC2258 prise au 46^{ième} passages à des souris sans défense immunitaire ("nude") selon le protocole décrit par Stauffer et al. (ci-dessus). Aucune formation de tumeur n'est visible après plus de 8 mois. Il est à noter que que la tumorigénicité des lignées n'est pas révélateur d'une non-transformation cancéreuse de ladite lignée. En-effet, à titre d'exemple la lignée NMC456 décrite pas Stauffer et al. n'induit pas de tumeurs chez des souris nude, bien qu'elle exprime des marqueurs tumoraux caractéristiques de sa transformation cancéreuse.

### 3. Expression de marqueurs tumoraux dans la lignée DSM ACC2258

On détermine selon le protocole décrit par Stauffer et al. si la lignée DSM ACC2258 exprime les marqueurs tumoraux suivants: la mutation p-53, l'antigène carcinoembryonnaire (CEA) et le gène de l'adénome du colyposis coli (APC).

Les résultats sont négatifs pour tous ces marqueurs, ce qui indique un état de non-transformation cancéreuse de la lignée.

### 4. Contamination virale de la lignée DSM ACC2258

On détermine si la lignée DSM ACC2258 est contaminée par le virus de l'hépatite C (HCV), le virus de l'hépatite B (HBV) et le virus du sida (HIV-1).

Pour l'analyse du HBV, on extrait de l'ADN à partir d'environ 2x10⁶ cellules par traitement dans des solutions de phénol et chloroforme suivi d'une précipitation dans l'éthanol. Des échantillons d'ADN sont alors soumis à une amplification par PCR en utilisant des amorces spéficiques de la région pre-core du virus (Lynch et al., J. Virol., 65, 3887-3890). On sépare ensuite les produits de l'amplification sur gel agarose, et on les visualise sous U.V. en présence de bromure d'éthidium. Pour comparaison, on analyse de la même manière en parrallèle une dilution d'un sérum contenant 10-10⁵ HBV (Anawa Biomedical Services 6 Product, USA).

Pour l'analyse du HIV-1, on soumet des échantillons d'ADN décrits ci-dessus soumis à une amplification par PCR en utilisant des amorces spéficiques de la région GAG du virus. On sépare ensuite les produits de l'amplification sur gel agarose, et on les visualise sous U.V. en présence de bromure d'éthidium. Pour comparaison, on analyse de la même manière en parrallèle une dilution d'un sérum contenant 10-10⁵ HIV-1 (Anawa Biomedical Services 6 Product, USA).

Pour l'analyse du HCV, on extrait l'ARN à partir d'environ 2x10⁶ cellules par la méthode de Chomczynski et al. (Anal. Biochem., 162, 156-159, 1987). On effectue classiquement une reverse-transcription, et on soumet l'ADN complémentaire obtenu à une amplification par PCR en utilisant des amorces spéficiques de la région 5' non-codante du virus. On sépare ensuite les produits de l'amplification sur gel agarose, et on les visualise sous U.V. en présence de bromure d'éthidium. Pour comparaison, on analyse de la même manière en parrallèle une dilution d'un sérum contenant 10-10⁵ HCV (Anawa Biomedical Services 6 Product, USA).

Les résultats sont négatifs pour la présence des 3 virus.

### 5. Expression de marqueurs spécifiques des cellules épithéliales humaines

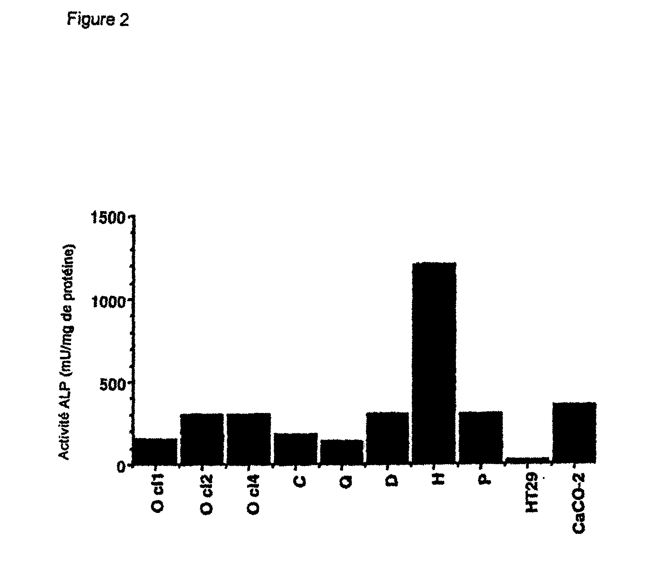

5.a. Cytokeratines: on fixe les cellules d'une culture de la lignée DSM ACC2258 sur plaques en verre par une solution de 100% méthanol froid, puis on lave les plaques dans un tampon comprenant 0,05M de Tris pH 8,6, 1,8% de NaCl et 0,2% de polyéthylène glycol 2000 (tampon TNP). On incube ensuite les cellules pendant 30 min en présence d'anticorps de souris spécifiques pour certaines cytokeratines (anti-CH peptide 4, 7, 8, 13, 14, 17, 18, 19, 20, Sigma, USA). Après 3 lavages dans le tampon TNP comprenant 0,5% de BSA, on incube les plaques pendant 30 min avec un anticorps de chèvre anti IgG de souris comprenant un composé immunofluorescent (1:300, FITC-goat anti mouse IgG; Biosys). Après 3 lavages dans le tampon précédent, on fixe les plaques et on les analyse par fluorescense sous microscopie. Toutes les cellules sont positives pour les cytokeratines 7, 8 et 17.
5.b. Vimentine: de la même manière que pour l'analyse des cytokeratines, on soumet les cellules fixées décrites ci-dessus à un anticorps de souris anti-vimentine (DAKO, USA), puis à l'anticorps de chèvre anti-IgG de souris mentionné ci-dessus. Sous microscopie par fluorescence, toutes les cellules sont positives pour la vimentine.
5.c. Connections entre les cellules: on cultive sur plaque de verre jusqu'à confluence les cellules de la lignée DSM ACC2258, on les fixe par traitement avec une solution de 2,5% de glutaraldéhyde dans un tampon phosphate 0,1 M pH 7,4 pendant 1 h, à température ambiante. Après deux lavages dans le même tampon phosphate, on fixe à nouveau les cellules dans une solution de 2% OsO₄ dans le même tampon. On déshydrate ensuite les cellules dans des solutions successives d'éthanol à 30, 50, 70, 90 et 100% (Polaron Equipment Ltd., Watford, UK), puis on recouvre les cellules par une fine couche d'or (SEM coating unit E5100, Polaron), On examine ensuite les cellules sous microscopie électronique (Philips 505 SEM). L'analyse révèle que les cellules présentent des connections inter-cellulaires qui sont caractéristiques des cellules épithéliales (en anglais "tight jonctions").
5.d. Phosphatase alcaline: on détermine l'activité phosphatase alcaline des cellules de la lignée DSM ACC2258 à l'aide du kit commercial Sigma qui reproduit la méthode décrite par Dahlquist et al. (Analytical Biochemistry, 22, 99-107, 1968). Les résultats présentés à la figure 2 montrent que les cellules de la lignée DSM ACC2258 (Ocl1, Ocl2, Ocl4) expriment une activité phosphatase comparable à celle des cellules épithéliales du colon humain cancéreuses CaCO-2, mais plus importante que des cellules épithéliales du colon humain cancéreuses HT-29. Les résultats C, Q, D, H, P concernent d'autres lignées épithéliales du colon humain présentées à l'exemple 2.
5.e. Cytochrome P450: On analyse l'expression de cytochromes par les cellules de la lignée DSM ACC2258, à l'aide de la technique connue RT-PCR en utilisant des amorces d'ADN spécifiques des différentes cytochromes P450. Ces amorces ont été préparées classiquement à partir de la séquence d'ADN des différentes cytochromes accessibles sur la base de donnée GeneBank (CYP1A1: n° accession X02612; CYP2C: n° accession M61855 ou M61858 ou M61856 ou MM61854; CYP2D6: n° accession M33388; CYP2E1: n° accession J02843; CYP3A5: n° accession J04813; CYP1A2: n° accession Z00036; CYP2A6: n° accession M33318; CYP2B6: n° accession M29874).
   Pour cela, on cultive les cellules jusqu'à confluence sur des boites de 35 mm (Costar), on extrait l'ARN à l'aide du kit RNAeasy (Qiagen), on effectue une reverse transcription (1st Strand cDNA Synthesis Kit for RT-PCR, Boehringer Mannheim), on soumet l'ADN complémentaire obtenu à une amplification par PCR en utilisant les amorces d'ADN spéficiques des différentes cytochromes P450, on sépare ensuite les produits de l'amplification sur gel d'agarose, et on les visualise sous U.V. en présence de bromure d'éthidium.
   Les résultats montrent que les cellules expriment les cytochromes CYP1A1, CYP2C, CYP2D6, CYP2E1, et CYP3A5. Par contre, les cellules sont négatives pour les cytochromes CYP1A2, CYP2A6 et CYP2B6. L'expression du cytochrome CYP3A5 est également confirmé par analyse western-blot d'un extrait de protéines des cellules DSM ACC2258 à l'aide d'un anticorps polyclonal anti-CYP3A5 (Oxygene, USA)
5.f. Enzymes impliquées dans l'oxydation cellulaire et la détoxification d'électrophiles: on cultive les cellules de la lignée DSM ACC2258, on extrait l'ARN par la méthode de Chomczynski et al. (Anal. Biochem., 162, 156-159, 1987), on prépare des sondes d'ADN à partir des séquences d'ADN de la Cu/Zn-superoxide dismutase (SOD), la glutathion péroxidase (GP), la catalase (CA), la glutathion-S-transférase (GST) et la quinone réductase (QR), qui sont accessibles sur la base de donnée GeneBank (GST: n° accession X08058; GP: n° accession M21304; CA: n° accession M81578; SOD: n° accession 729336; QR: n° accession M81596_600), puis on effectue un Nothern-blot de l'ARN avec ces sondes pour confirmer la transcription de ces enzymes.

Les résultats montrent que toutes les cellules expriment une transcription des gènes codant pour l'activité SOD, GP, CA, GST et QR. La lignée DSM ACC2258 exprime donc des enzymes impliquées dans l'oxydation cellulaire et la détoxification d'électrophiles.

### 6. Expression de marqueurs spécifiques des cellules épithéliales du colon humain

6.a. Villosités de surface: comme décrit au point 5.c ci-dessus, l'analyse par microscopie électronique des cellules montrent clairement la présence de villosités au pôle opposé de celui qui adhére au support.
6.b. Sucrose isomaltase: la sucrose-isomaltase est spécifique des cellules épithéliales du colon humain. On peut mettre en évidence la présence de sucrose-isomaltase au niveau de l'ARN messager par la technique RT-PCR, décrite plus haut au point 5.e, après exposition d'une culture confluente de cellules DSM ACC2258 pendant 48 h à 1 ng/ml de TGF-β recombinant humain (Becton Dickinson). Les amorces d'ADN sont préparées classiquement à partir de la séquence d'ADN du gène de la sucrose-isomaltase accessible sur la banque de données GeneBank (n° accession X63597 S41833 S41836).
6.c. Dipeptidylpeptidase IV: la DPPIV est spécifique des cellules épithéliales du colon humain. On peut mettre en évidence sa présence au niveau de l'ARN messager par la technique RT-PCR, décrite plus haut au point 5.e. Les amorces d'ADN sont préparées classiquement à partir de la séquence d'ADN du gène de la DPPIV accessible sur la banque de données GeneBank (n° accession U13710-35)
6.d. Complexe maieur d'histocompatibilité de classe II: les cellules épithéliales du colon peuvent exprimer le complexe majeur d'histocompatibilité de classe II (antigènes HLA) en présence d'interféron γ (IFNγ). Pour cela, on cultive les cellules DSM ACC2258 dans le milieu B50, en présence de 100U/ml d'IFNγ (Boehringer Mannheim) recombinant à 12, 24, 36 et 48 h de culture. On sépare les cellules par une solution comprenant 0,025% de trypsine et de l'EDTA (Gibco Life Technologie, USA), on les incube en présence d'anticorps spécifiques pour les antigènes HLA-DR, HLA-DP et HLA -DQ (Becton-Dickinson, USA), et on les sépare par un cytomètre de flux (FACS can, Becton-Dickinson).

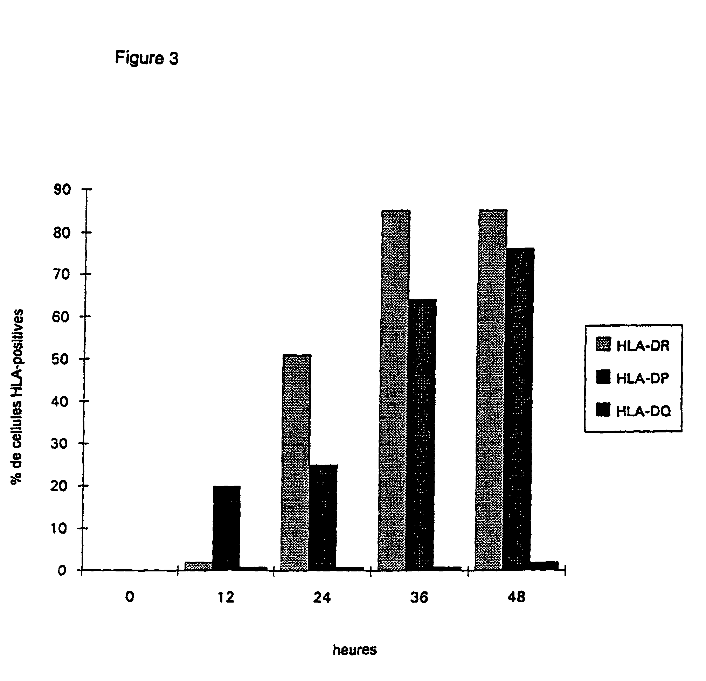

Les résultats présentés à la figure 3 montrent une expression de l'antigène HLA-DP dès 12 h de culture, avec des concentrations croissantes à 24 et 48 h. L'expression de HLA-DR est observé après 24 h de culture avec des concentrations maximales à 36 et 48 h. Par contre, l'antigène HLA-DQ n'est pas induit ce qui confirme les observations faites sur les cellules primaires épithéliales du colon (Mayer et al.).

### 7. Adhésion à la souche de bactérie CNCM-1225

7.a Adhésion: l'intestin humain est colonisé par de nombreuses bactéries non-pathogènes, parmi lesquelles on peut compter les lactobacilles et les bifidobactéries, dont certains sont capables d'adhérer aux villosités de la muqueuse intestinale. On cultive ainsi pendant 12h la souche de bactérie CNCM-1225, connue pour fortement adhérer aux cellules épithéliales du tractus intestinal (EP577904; Bernet et al.), dans un milieu MRS (Man, Rogosa and Sharpe, Biokar) comprenant 100µC de ³H-adenine, en absence d'oxygène.
On cultive les cellules DSM ACC2258 pendant 2 semaines jusu'à confluence, puis on les incube pendant 1h à 37°C avec le milieu MRS de 12h décrit ci-dessus, ou un milieu frais MRS ou un tampon phosphate pH 6,5 comprenant 4x10⁸ cellules par ml issues de la culture MRS de 12h décrite ci-dessus. On lave 3 fois les cellules DSM ACC2258 avec du PBS, on les lyse avec une solution de NaOH 1M, puis on mesure l'intensité radioactive dégagée par les cellules à l'aide d'un compteur à scintillation.
Pour comparaison, de la même manière que décrit ci-dessus, on cultive la souche CaCO-2, on l'incube avec la souche CNCM I-1225 et on mesure l'intensité radioactive dégagée par les cellules.
Les résultats présentés dans le tableau 2 ci-après, montrent que les cellules DSM ACC2258 sont capables d'adhérer les bactéries CNCM I-1225, aussi bien que les cellules CaCO-2. On peut également noter que la culture de la souche LA-1 de 12 h contient manifestement des facteurs qui favorisent l'adhésion des bactéries. En-effet, le milieu frais MRS dans lequel on a ajouté des bactéries présente un niveau d'adhésion moins élevé. De plus, on peut observer qu'un pH de 6,5, qui est similaire à celui retrouvé dans le tractus intestinal *in-vivo*, favorise également l'adhésion des bactéries, lorsque l'on compare au niveau d'adhésion obtenu avec le milieu frais MRS présentant un pH de l'ordre de 5.

**Tableau 2**

| Lignée cellulaire | Conditions d'incubations | % ³H-adenine |
|---|---|---|
| DSM ACC2258 | MRS de 12 h | 30,2 |
| | MRS frais | 11,8 |
| | Tampon pH 6,5 | 13,03 |
| CaCo-2 | MRS de 12 h | 34,8 |
| | MRS frais | 6,1 |
| | Tampon pH 6,5 | 8,3 |

7.b Nombre de bactéries adhérantes
On mesure la capacité de la lignée DSM ACC2258 a retenir les bactéries CNCM I-1225 selon la méthode décrite par Bernet et al. (Gut, 35, 483-489, 1994). En résumé, on détermine visuellement le nombre de bactéries qui adhérent à 100 cellules du colon. Pour cela, on utilise un milieu de culture MRS de 12h, ou un milieu frais MRS comprenant 4x10⁸ cellules par ml issues de cette culture MRS de 12h. De plus, on effectue l'analyse sur trois préparations, et on utilise 20 vues microscopiques prises au hasard dans chaque préparation.
Pour comparaison, on mesure également de la même manière la capacité de la lignée CaCO-2 a retenir les bactéries CNCM I-1225.
Les résultats présentés dans le tableau ci-dessous montre que la lignée est capable de retenir un nombre significatif de bactéries. On peut également noter que la culture de la souche LA-1 de 12 h contient manifestement des facteurs qui favorisent l'adhésion des bactéries. En-effet, le milieu frais MRS dans lequel on a ajouté des bactéries ne permet pas d'obtenir un niveau d'adhésion aussi élevé.

**Tableau 3**

| Lignée cellulaire | Conditions d'incubations | Nb / 100 cellules | Déviation standart |
|---|---|---|---|
| DSM ACC2258 | MRS de 12 h | 118 | 7 |
| | MRS frais | 13 | 4 |
| CaCo-2 | MRS de 12 h | 124 | 13 |
| | MRS frais | 9 | 4 |

### EXAMPLE 2

Les autres lignées immortalisées de cellules épithéliales du colon humain développées à l'exemple 1, présentent des caractéristiques similaires à celles décrites plus haut pour la souche DSM ACC2258. A titre d'exemple, l'activité phosphatase alcaline de 5 autres lignées appellées C, Q, D, H, P sont présentées à la figure 2.

## Revendications

1. Milieu de culture sans sérum adaptée à la culture et à l'immortalisation de cellules épithéliales du colon humain, comprenant des sels inorganiques, du glucose, du pyruvate de sodium, des acides aminés, de la BSA, de l'éthanolamine, de la phosphoéthanolamine, des vitamines, et des hormones **caractérisé en ce que** le milieu comprend des traces d'oligoélements, des vitamines consistant en la vitamine C et l'acide rétinoique, et des hormones consistant en la triiodothyrodine, la déxamethasone, l'hydrocortisone, l'extrait de la glande pituaire bovine, l'insuline, l'EGF et la transférrine.

2. Milieu de culture selon la revendication 1, **caractérisé en ce que** le milieu comprend 1-100 nM d'oligoéléments, 10-1000 nM d'acide rétinoique, 10-1000 nM de triiodothyrodine, 0,1-50 nM de déxamethasone, 1-100 nM d'hydrocortisone, 1-100 µg/ml de vitamine C, 1-100 µg/ml d'extrait de la glande pituaire bovine, 0,1-50 µl/ml d'insuline, 0,1-50 ng/ml l'EGF et 0,1-100 µg/ml la transférrine.

3. Milieu de culture selon la revendication 1, **caractérisé en ce que** les sels inorganiques sont choisis dans le groupe formé par le chlorate de sodium, le chlorate de potassium, le sulfate de potassium, le chlorate de calcium, le phosphate de sodium di-basique, le bicarbonate de sodium, le nitrite ferrique, le métévanadate d'ammonium, le molybdate d'ammonium, le sulfate cuprique, le chlorate de magnésium, le chlorate de manganèse, le sulfate de nickel, l'acétate de sodium, le pyruvate de sodium, le sélénite de sodium, le silicate de sodium, le chlorate d'étain, et le sulfate de zinc.

4. Milieu de culture selon la revendication 1, dans lequel les vitamines sont choisies dans le groupe formé par le D-pantothénate de calcium, le chlorate de choline, l'acide folique, l'inositol, la nicotinamide, la pyridoxine, la riboflavine, la thiamine, la biotine et le cyanocobalamine.

5. Milieu de culture selon la revendication 1, **caractérisé en ce que** le milieu comprend moins de 80 µM de calcium.

6. Milieu de culture selon la revendication 1, **caractérisé en ce qu'**il présente la composition B50 définie au tableau 1.

7. Procédé d'immortalisation de cellules épithéliales du colon humain, dans lequel on prépare une culture de cellules épithéliales primaires issues du colon humain, on infecte la culture avec un virus recombinant, on cultive les cellules immortalisées dans le milieu de culture sans sérum selon l'une des revendication 1 à 6.

8. Procédé selon la revendication 7, dans lequel on cultive les cellules épithéliales primaires dans le milieu de culture sans sérum selon l'une des revendications 1 à 6.

9. Procédé selon la revendication 7, dans lequel on infecte la culture avec un virus recombinant SV40 T-Ag contenant une partie du rétrovirus MuLV.

10. Procédé pour identifier l'effet mutagène, toxique ou bénéfique d'un agent sur le métabolisme des cellules du tractus intestinal, dans lequel, *in-vitro*, (1) on fait réagir, on cultive ou on met en contact avec une culture comprenant une lignée cellulaires selon l'une des revendications 11 à 16, un agent soupçonné d'être un agent mutagène, toxique ou bénéfique pour le métabolisme des cellules du tractus intestinal, et (2) on détermine ou on mesure les effets dudit agent sur ladite lignée cellulaire.

11. Lignée de cellules épithéliales du colon humain, immortalisée à l'aide d'un virus recombinant, la dite lignée étant
- capable de se multiplier durant au moins 60 passages successifs,
- dépourvue de marqueurs tumoraux,
- exprimant des marqueurs métabolique spécifiques des cellules épithéliales humaines non-immortalisées et des marqueurs métaboliques de différenciation spécifiques des cellules épithéliales non-immortalisées du colon humain,
- capable d'adhérer *in-vitro* à la souche de bactérie lactique CNCM I-1225, et
- capable d'être obtenue selon un procédé d'une des revendications 7 à 10,
sous réserve que les lignées de cellules soient exclues qui sont obtenues par un procédé pour obtenir une lignée immortalisée de cellules épithéliales humaines substantiellement pure, comprenant :
(a) mettre à disposition une culture primaire de cellules épithéliales humaines obtenue en utilisant un système de co-culture de fibroblastes, comprenant une première population de cellules comprenant une culture de fibroblastes humains sur un substrat dans un milieu approprié, un support du type d'une membrane, le support étant arrangé sur la culture de fibroblastes et une deuxième, population hétérogène de cellules appliquée sur le support et comprenant des cellules épithéliales humaines,
(b) ensemencer sur une plaque une troisième population de cellules comprenant une culture de fibroblastes humains sur un deuxième substrat dans un milieu approprié,
(c) détacher sélectivement des cellules non-épithéliales du substrat en incubant la co-culture avec un agent détachant approprié pendant une période de temps suffisante pour détacher des cellules non-épithéliales du substrat sans détacher une quantité substantielle de cellules épithéliales,
(d) arranger le support sur la culture des fibroblastes obtenue par l'étape (b), afin qu'une souche de cellules épithéliales substantiellement pure soit maintenue sur le support, et
(e) immortaliser ladite souche de cellules épithéliales substantiellement pure avec au moins un vecteur rétroviral ayant une cassette d'expression comprenant un molécule d'acide nucléique codant pour un polypeptide de papillomavirus choisi dans le groupe formé par E6, E7 et par des combinaisons de ceux-ci, et des séquences de contrôle qui dirigent la transcription du molécule d'acide nucléique, afin que la séquence puisse être transcrite et traduite dans la lignée cellulaire transfectée.

12. Lignée immortalisée selon la revendication 11, exprimant comme marqueur métabolique spécifique des cellules épithéliales humaines non-immortalisées au moins deux marqueurs choisis dans le groupe formé par la vimentine, des cytokeratines, des connections entre les cellules, la phosphatase alcaline, des cytochromes P450, une glutathion-S-transférase, la quinone réductase, la Cu/Zn-superoxide dismutase, la glutathion péroxidase et une catalase.

13. Lignée immortalisée selon la revendication 11, exprimant comme marqueur métabolique de différenciation au moins deux marqueurs choisis dans le groupe formé par les villosités de surface, la sucrose isomaltase, les complexes majeurs d'histocompatibilités de classe II, et la dipeptidylpeptidase IV.

14. Lignée immortalisée selon la revendication 13, exprimant les antigènes HLA-DR et HLA-DP du complexe majeur d'histocompatibilité de classe II, et n'exprimant pas l'antigène HLA-DQ dudit complexe, en présence d'interféron gamma humain.

15. Lignée immortalisée selon la revendication 11, capable d'adherer *in-vitro* la souche de bactérie lactique CNCM I-1225 à raison d'au moins 80 bactéries pour 100 cellules épithéliales du colon.

16. Lignée immortalisée selon la revendication 11, présentant le numéro de dépot DSM ACC2258.

17. Kit de diagnostique comprenant les cellules épithéliales immortalisées du colon humain selon l'une des revendications 11 à 16, le milieu de culture selon l'une des revendications 1 à 6, et des réactifs pour déterminer une réponse métaboliques desdites cellules à des agents mutagéniques, toxiques ou bénéfiques pour le métabolisme des cellules du tractus intestinal.

18. Utilisation des cellules épithéliales immortalisées du colon humain selon l'une des revendications 11 à 16, dans des procédés *in-vitro* d'identification d'agents mutagéniques, toxiques ou bénéfiques pour le métabolisme des cellules du tractus intestinal.

19. Utilisation des cellules épithéliales immortalisées du colon humain selon l'une des revendications 11 à 16 pour la préparation d'un agent pharmaceutique actif.

## Patentansprüche

1. Kulturmedium ohne Serum, geeignet zur Kultivierung und Immortalisierung von Epithelzellen des humanen Kolons, welches anorganische Salze, Glukose, Natriumpyruvat, Aminosäuren, BSA (Bovines Serum Albumin), Ethanolamin, Phoshoethanolamin, Vitamine und Hormone enthält, **dadurch gekennzeichnet, dass** das Medium Spuren von Spurenelementen, von Vitaminen, bestehend aus Vitamin C und Retinolsäure, und von Hormonen, bestehend aus Trijodthyroidin, Dexamethason, Hydrokortison, einem Extrakt aus der Rinderhirnanhangdrüse, Insulin, EGF und Transferrin, enthält.

2. Kulturmedium nach Anspruch 1, **dadurch gekennzeichnet, dass** das Medium 1-100 nM Spurenelemente, 10-1000 nM Retinolsäure, 10-1000 nM Trijodthyroidin, 0,1-50 nM Dexamethason, 1-100 nM Hydrokortison, 1-100 µg/ml Vitamin C, 1-100 µg/ml Extrakt aus der Rinderhirnanhangdrüse, 0,1-50 µl/ml Insulin, 0,1-50 ng/ml EGF und 0,1-100 µg/ml Transferrin enthält.

3. Kulturmedium nach Anspruch 1, **dadurch gekennzeichnet, dass** die anorganischen Salze aus der Gruppe ausgewählt werden, bestehend aus Natriumchlorat, Kaliumchlorat, Kaliumsulfat, Kalziumchlorat, Natriumdihydrogenphosphat, Natriumbicarbonat, Eisenferrit, Ammoniummetavanadat, Ammoniummolybdat, Kupfersulfat, Magnesiumchlorat, Manganchlorat, Nickelsulfat, Natriumacetat, Natriumpyruvat, Natriumselenit, Natriumsilikat, Zinnchlorat und Zinksulfat.

4. Kulturmedium nach Anspruch 1, in welchem die Vitamine aus der Gruppe ausgewählt werden, bestehend aus D-Kalziumpantothenat, Cholinchlorat, Folsäure, Inositol, Nicotinamid, Pyridoxin, Riboflavin, Thiamin, Biotin und dem Cyanocobalamin.

5. Kulturmedium nach Anspruch 1, **dadurch gekennzeichnet, dass** das Medium weniger als 80 µM Kalzium enthält.

6. Kulturmedium nach Anspruch 1, **dadurch gekennzeichnet, dass** es die in der Tabelle 1 definierte Zusammensetzung B50 darstellt.

7. Verfahren zur Immortalisierung von Epithelzellen des humanen Kolons, in welchem eine Primärkultur von Epithelzellen aus humanem Kolon hergestellt wird, die Kultur mit einem rekombinanten Virus infiziert wird, die immortalisierten Zellen in einem Kulturmedium ohne Serum nach einem der Ansprüche 1 bis 6 kultiviert wird.

8. Verfahren nach Anspruch 7, in welchem die primären Epithelzellen in dem Kulturmedium ohne Serum gemäß einem der Ansprüche 1 bis 6 kultiviert wird.

9. Verfahren nach Anspruch 7, in welchem die Kultur mit einem rekombinanten Virus SV40 T-Ag, infiziert wird, welcher einen Teil des Retrovirus MuLV enthält.

10. Verfahren zur Identifikation des Effektes eines Agens, das mutagen, toxisch oder fördernd für den Metabolismus von Zellen des Verdauungstraktes ist, in welchem man *in vitro* (1) auf eine Kultur, die eine Zelllinie nach einem der Ansprüche 11 bis 16 enthält, ein Agens, von dem angenommen wird, dass es ein Agens ist, das mutagen, toxisch oder förderlich für den Metabolismus von Zellen des Verdauungstraktes ist, einwirken lässt, es mit einer solchen Kultur kultiviert oder mit einer solchen Kultur in Kontakt bringt, und (2) die Effekte des besagten Agens auf die besagte Zelllinie bestimmt oder misst.

11. Linie von Epithelzellen des humanen Kolons, immortalisiert mit Hilfe eines rekombinanten Virus, wobei die besagte Zelllinie
- in der Lage ist, sich während mindestens 60 aufeinaderfolgender Passagen zu vermehren,
- keine Tumormarker aufweist,
- die Marker, die für humane, nicht immortalisierte Epithelzellen spezifisch sind, und Differenzierungsmarker, die für nicht immortalisierte Epithelzellen des humanen Kolons spezifisch sind, exprimiert,
- in der Lage ist, *in vitro* an den Milchsäurebakterienstamm CNCMI-1225 zu binden, und
- durch ein Verfahren nach einem der Ansprüche 7 bis 10 gewinnbar ist,
vorausgesetzt, dass es sich nicht um eine Zelllinie handelt, die durch ein Verfahren zur Gewinnung einer immortalisierten Linie von im Wesentlichen reinen humanen Epithelzellen gewonnen wurde, wobei dieses Verfahren folgende Schritte aufweist:
(a) zur Verfügung stellen einer Primärkultur von humanen Epithelzellen, die durch ein System der Kokultivierung von Fibroblasten gewonnen wurde, welche eine erste Population von Zellen enthält, welche eine Kultur humaner Fibroblasten auf einem Substrat in einem geeigneten Medium enthält, einen Support vom Typ einer Membran, wobei der Support über der Kultur der Fibroblasten angeordnet ist, und eine zweite, heterogene Population von Zellen, die auf dem Support aufgebracht ist, welche humane Epithelzellen enthält,
(b) Aussähen auf einer Kulturschale einer dritten Population von Zellen, welche eine Kultur humaner Fibroblasten auf einem zweiten Substrat in einem geeigneten Medium enthält,
(c) selektives Ablösen von nicht-epithelialen Zellen von dem Substrat durch Inkubation der Kokultur mit einem geeigneten ablösenden Agens, während einer Zeitspanne, die ausreicht, um nicht-epitheliale Zellen von dem Substrat der Kokultur abzulösen, ohne einen erheblichen Teil der epithelialen Zellen abzulösen,
(d) Anordnen des Supports auf der Fibroblastenkultur, welche durch Schritt (b) erhalten wurden, damit ein Stamm von im Wesentlichen reinen Epithelzellen auf dem Support erhalten wird, und
(e) Immortalisieren des besagten Stammes von im Wesentlichen reinen epithelialen Zellen mit mindestens einem retroviralen Vektor, der eine Expressionskassette aufweist, die ein Nukleinsäuremolekül enthält, das für ein Polypeptid von Papillomavirus kodiert ist, ausgewählt aus der Gruppe bestehend aus E6, E7 und Kombinationen davon, und Kontrollsequenzen, welche die Transkription des Nukleinsäuremoleküls steuern, damit die Sequenz in der transfizierten Zelllinie transkribiert und übersetzt werden kann.

12. Immortalisierte Linie nach Anspruch 11, die als Marker, der für humane, nichtimmortalisierte Epithelzellen spezifisch ist, mindestens zwei Marker exprimiert, ausgewählt aus der Gruppe bestehend aus Vimentin, Cytokeratinen, Zell-Zell Verbindungen, alkalischer Phosphatase, P450 Zytochromen, einer Glutathion-S-transferase, der Chinonreduktase, der Cu/Zn-Superoxiddismutase, der Glutathionperoxidase und einer Katalase.

13. Immortalisierte Linie nach Anspruch 11, die als Differenzierungsmarker mindestens zwei Marker ausgewählt aus der Gruppe bestehend aus Oberflächenvilli, Sucrose isomaltase, Major Histocompatibility Complex II (MHC II) und der Dipepitidylpeptidase IV exprimiert.

14. Immortalisierte Linie nach Anspruch 13, welche in Anwesenheit von humanem Interferon-gamma die Antigene HLA-DR und HLA-DP des Major Histocompatibility Complex II (MHC II) exprimiert, und nicht das Antigen HLA-DQ des besagten Komplexes exprimiert,

15. Immortalisierte Linie nach Anspruch 11, die in der Lage ist, im Verhältnis von mindestens 80 Bakterien zu je 100 Epithelzellen des Kolons, *in-vitro* an den Milchsäurebakterienstamm CNCM I-1225 zu binden.

16. Immortalisierte Linie gemäß Anspruch 11, hinterlegt unter Depotnummer DSM ACC2258.

17. Diagnostischer Kit, welcher immortalisierte Epithelzellen des humanen Kolons nach einem der Ansprüche 11-16, das Kulturmedium nach einem der Ansprüche 1 bis 6, und Reagenzien zur Bestimmung einer Stoffwechselreaktion besagter Zellen auf Agenzien, die mutagen, toxisch, oder förderlich für den Stoffwechsel von Zellen des Verdauungstraktes sind, enthalten.

18. Verwendung von immortalisierten Epithelzellen des humanen Kolons nach einem der Ansprüche 11 bis 16, in den *in vitro* Verfahren zur Identifikation von Agenzien, die mutagen, toxisch, oder förderlich für den Stoffwechsel von Zellen des Verdauungstraktes sind.

19. Verwendung von immortalisierten Epithelzellen des humanen Kolons nach einem der Ansprüche 11 bis 16 zur Herstellung eines pharmazeutisch aktiven Agens.

## Claims

1. Serum-free culture medium, suitable for culturing and immortalizing human colon epithelial cells, comprising inorganic salts, glucose, sodium pyruvate, amino acids, BSA, ethanolamine, phosphoethanolamine, vitamins and hormones, **characterized in that** the medium comprises trace elements, vitamins consisting of vitamin C and retinoic acid, and hormones consisting of triiodothyrodine, dexamethasone, hydrocortisone, bovine pituitary gland extract, insulin, EGF and transferrin.

2. Culture medium according to Claim 1, **characterized in that** the medium comprises 1-100 nM of trace elements, 10-1 000 nM of retinoic acid, 10-1 000 nM of triiodothyrodine, 0.1-50 nM of dexamethasone, 1-100 nM of hydrocortisone, 1-100 µg/ml of vitamin C, 1-100 µg/ml of bovine pituitary gland extract, 0.1-50 µg/ml of insulin, 0.1-50 ng/ml of EGF and 0.1-100 µg/ml of transferrin.

3. Culture medium according to Claim 1, **characterized in that** the inorganic salts are chosen from the group made up of sodium chlorate, potassium chlorate, potassium sulphate, calcium chlorate, dibasic sodium phosphate, sodium bicarbonate, ferric nitrite, ammonium metavanadate, ammonium molybdate, cupric sulphate, magnesium chlorate, manganese chlorate, nickel sulphate, sodium acetate, sodium pyruvate, sodium selenite, sodium silicate, tin chlorate and zinc sulphate.

4. Culture medium according to Claim 1, in which the vitamins are chosen from the group made up of D-calcium pantothenate, choline chlorate, folic acid, inositol, nicotinamide, pyridoxine, riboflavin, thiamine, biotin and cyanocobalamine.

5. Culture medium according to Claim 1, **characterized in that** the medium comprises less than 80 µM of calcium.

6. Culture medium according to Claim 1, **characterized in that** it has the composition B50 defined in Table 1.

7. Method for immortalizing human colon epithelial cells, in which a culture of primary epithelial cells derived from human colon is prepared, the culture is infected with a recombinant virus, and the immortalized cells are cultured in the serum-free culture medium according to one of Claims 1 to 6.

8. Method according to Claim 7, in which the primary epithelial cells are cultured in the serum-free culture medium according to one of Claims 1 to 6.

9. Method according to Claim 7, in which the culture is infected with a recombinant SV40 T-Ag virus containing part of the MuLV retrovirus.

10. Method for identifying the mutagenic, toxic or beneficial effect of an agent on the metabolism of cells of the intestinal tract, in which, *in vitro,* (1) an agent suspected of being an agent which is mutagenic, toxic or beneficial with respect to the metabolism of cells of the intestinal tract is reacted, cultured or brought into contact with a culture comprising a cell line according to one of Claims 11 to 16, and (2) the effects of said agent on said cell line are determined or measured.

11. Human colon epithelial cell lines immortalized using a recombinant virus, the said line
- being capable of multiplying for at least 60 successive passages,
- lacking tumour markers,
- expressing metabolic markers specific for non-immortalized human epithelial cells and metabolic differentiation markers specific for non-immortalized human colon epithelial cells,
- being capable of adhering, *in vitro,* to the lactic acid bacterial strain CNCM I-1225, and
- being able to be obtained according to a method of one of Claims 7 to 10,
with the proviso that this excludes the cell lines which are obtained using a process for obtaining a substantially pure immortalized human epithelial cell line, comprising:
(a) providing a primary culture of human epithelial cells obtained using a fibroblast co-culture system comprising a first population of cells comprising a culture of human fibroblasts on a substrate in a suitable medium, a support such as a membrane, the support being arranged over the fibroblast culture, and a second, heterogeneous population of cells applied to the support and comprising human epithelial cells,
(b) plating a third population of cells comprising a culture of human fibroblasts onto a second substrate in a suitable medium,
(c) selectively detaching non-epithelial cells from the substrate by incubating the co-culture with a suitable detaching agent for a period of time sufficient to detach non-epithelial cells from the substrate without detaching a substantial amount of epithelial cells,
(d) arranging the support over the fibroblast culture obtained in step (b), such that a substantially pure epithelial cell strain is maintained on the support, and
(e) immortalizing said substantially pure epithelial cell strain with at least one retroviral vector having an expression cassette comprising a nucleic acid molecule encoding a papillomavirus polypeptide chosen from the group made up of E6 and E7 and combinations thereof, and control sequences which direct the transcription of the nucleic acid molecule, such that the sequence may be transcribed and translated in the transfected cell line.

12. Immortalized line according to Claim 11, expressing, as metabolic marker specific for non-immortalized human epithelial cells, at least two markers chosen from the group made up of vimentin, cytokeratins, connections between cells, alkaline phosphatase, cytochrome P450s, a glutathione-S-transferase, quinone reductase, Cu/Zn-superoxide dismutase, glutathione peroxidase and a catalase.

13. Immortalized line according to Claim 11, expressing, as metabolic differentiation marker, at least two markers chosen from the group made up of surface villi, sucrose-isomaltase, class II major histocompatibility complexes and dipeptidyl peptidase IV.

14. Immortalized line according to Claim 13, expressing the HLA-DR and HLA-DP antigens of the class II major histocompatibility complex, and not expressing the HLA-DQ antigen of said complex, in the presence of human gamma interferon.

15. Immortalized line according to Claim 11, capable of adhering, *in vitro,* to the lactic acid bacterial strain CNCM I-1225 in a proportion of at least 80 bacteria per 100 colon epithelial cells.

16. Immortalized line according to Claim 11, having the deposit number DSM ACC2258.

17. Diagnostic kit, comprising the immortalized human colon epithelial cells according to one of Claims 11 to 16, the culture medium according to one of Claims 1 to 6, and reagents for determining a metabolic response of said cells to agents which are mutagenic, toxic or beneficial with respect to the metabolism of cells of the intestinal tract.

18. Use of the immortalized human colon epithelial cells according to one of Claims 11 to 16, in methods for identifying, *in vitro,* agents which are mutagenic, toxic or beneficial with respect to the metabolism of cells of the intestinal tract.

19. Use of the immortalized human colon epithelial cells according to one of Claims 11 to 16, for preparing an active pharmaceutical agent.
